# EUROPEAN PATENT SPECIFICATION

(11) **EP 0 569 992 B1**
(45) Date of publication and mention of the grant of the patent: **13.09.1995**
(21) Application number: 93107835.6
(22) Date of filing: 13.05.1993
(51) Int. Cl.: C07D 303/48, C07B 57/00

(54) **Method for the preparation of optically active derivatives of epoxypropionic acid**
Verfahren zur Herstellung von Derivaten der epoxypropionsäure
Procédé pour la préparation de dérivés de l'acide époxypropionique

(30) Priority: 14.05.1992 FI 922189
(43) Date of publication of application: 18.11.1993
(73) Proprietor: ORION-YHTYMÄ OY FERMION, 02200 Espoo (FI)
(72) Inventor: Aaltonen, Olli, SF-00250 Helsinki (FI); Komppa, Veikko, SF-03100 Nummela (FI); Hase, Anneli, SF-02500 Helsinki (FI); Kairisalo, Pekka, SF-00950 Helsinki (FI); Hytönen, Martti, SF-02600 Espoo (FI); Alkio, Martti, SF-02170Espoo (FI)
(74) Representative: Barz, Peter, Dr.

(56) References cited:
- EP-A- 0 342 903
- JOURNAL OF CHROMATOGRAPHY vol. 371 , 26 December 1986 , AMSTERDAM, NL pages 153 - 158 S. HARA ET AL. 'Carbon dioxide supercritical fluid chromatography on a chiral diamide stationary phase for the resolution of D- and L-amino acid derivatives'
- JOURNAL OF CHROMATOGRAPHIC SCIENCE vol. 27, no. 7 , 1989 , NILES, ILL. US pages 383 - 394 P. MACAUDIERE ET AL. 'Carbon dioxide supercritical fluid chromatography with chiral stationary phases: a promising coupling for the resolution of various racemates'

## Description

The present invention relates to a method for the preparation of optically active derivatives of epoxypropionic acid.

The derivatives of epoxypropionic acid are important intermediates in the preparation of some drugs, for example 1,5-benzothiazepine. The derivatives of epoxypropionic acid can appear as different optically active space structures. Usually only one of the optically active isomers of the final drug exhibits the desired effect. The other optical isomers are either ineffective or they may have harmful side effects. For that reason it is appropriate to aim to use and manufacture the drugs as optically pure isomers.

Macaudiére, P. et al. (Journal of Chromatographic Science, 27, 383-394, 1989) in general review the use of chiral stationary phases (CSPs) in supercritical fluid chromatography with carbon dioxide, in particular the resolution of optical isomers on various Pirkle-type CSPs, cyclodextrin CSPs and polymer CSPs.

The EP-A-342 903 discloses the separation of the enantiomers of 3-(4-methoxyphenyl)-epoxypropionic acid from each other by hydrolysing first the ester of the racemic crude material and bringing the deglycidate thus obtained in contact with an optically active amine. The obtained diastereomeric salt is crystallized as optically pure, converted to an alkali metal salt and alkylated to an optically pure ester. EP-A-386 654, JP 61-145159 and JP 61-145160 also describe corresponding resolution methods based on the formation of diastereomeric salts. These methods contain several steps, they take many hours and various reagents and solvents are required therein. They produce plenty of waste solutions, which are expensive to purify and finally need to be destroyed.

The WO 89/10350 discloses the synthesis of the desired optically active derivative of epoxypropionic acid by way of a sulfonate ester intermediate. This method also involves numerous reagents and vast amounts of different solvents, which need to be purified for reuse and the destruction of which has to be taken care for.

The EP-A-365 029 describes the synthesis of optically active epoxypropionic acids from the racemic dihalopropionic acid or chlorolactic acid by using as catalyst dehalogenase enzyme, that is separated from a culture of a Pseudomonas geneva population. The optically pure synthesis product is finally crystallized from the reaction mixture in several steps using many solvents. The enzymatically catalyzed synthesis requires a long reaction time, at least 12 hours, so the manufacturing rate of the method is very low.

The EP-A-362 556, EP-A-343 714 and WO 90/04643 describe the separation of the enantiomers of a mixture of racemic epoxypropionic acid from each other by hydrolyzing their esters stereospecifically using enzymes, particularly lipases, as catalysts. Also these methods require very long reaction times, up to 48 hours. Many very dilute solutions, purification, regeneration, destruction and handling of the waste water of which are expensive, are used in the methods for separation of enantiomers based on enzymatic hydrolysis.

It is also known to synthesize optically active epoxypropionic acids or their derivatives by using L-aminoacids as starting material (JP 62-212329) or other optically pure starting materials (JP 60-13776, JP 60-13775). The method for synthesis involves many steps and many crystallizations from different solvents. Several reagents are used and lots of waste solutions are produced.

Surprisingly we have noticed, that the enantiomers of the derivatives of epoxypropionic acid can simply be separated from each other by dissolving the racemic mixture to be separated in carbon dioxide and passing the obtained solution through a chromatographic column.

The present invention therefore relates to a method for preparing optically pure derivatives of epoxypropionic acid having the general formula 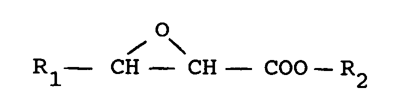
wherein R₁ and R₂, which may be the same or different from each other, are H, an alkyl group or a substituted or unsubstituted phenyl group, from racemic mixtures thereof, comprising the following steps:
a) dissolving the racemic mixture to be separated in carbon dioxide,
b) passing the obtained solution through a chromatographic column, and
c) separating the desired optical isomer of epoxypropionic acid from the eluant.

The alkyl group preferably has 1 to 4 carbon atoms. Examples for alkyl are methyl, ethyl, propyl, butyl, iso-butyl, and tert.-butyl.

The phenyl group may be substituted, for instance, by an alkoxy group, preferably having 1 to 4 carbon atoms. Examples for alkoxy are methoxy, ethoxy, propoxy, butoxy and tert.-butoxy.

The separation of enantiomers of the derivatives of epoxypropionic acid chromatographically by using carbon dioxide as mobile phase enables a simple, fast and, having regard to occupational and environmental safety, a clean industrial preparation process for optically pure enantiomers of epoxypropionic acid.

One of the advantages of this invention is that the separation of enantiomers of the derivatives of epoxypropionic acid can be performed stepwise and continuously, so that the time needed for separating one batch is short. The next batch of racemic mixture to be separated can be charged to the chromatographic column soon after the previous one so that the eluting, optically pure enantiomers will not get mixed to it. The batches to be separated can thus be typically charged to the chromatographic column at intervals of a few minutes. This accelerates the purification process considerably in comparison with known methods. The benefit from a large production rate is a drastic reduction of equipment size.

It is a further advantage of this invention that the optical purity of the obtained enantiomers can be almost freely chosen and can be optimized according to the requirements of the quality of the product and the economy.

Moreover it is an advantage, that the whole preparation process is simple. It contains only three main steps: dissolving the racemic mixture in carbon dioxide, chromatographic separation of the enantiomers, and separation of the optically pure product from carbon dioxide. No reagents are needed and the whole purification process can be performed using only one solvent, carbon dioxide. Using only one solvent results in a significant reduction of costs.

One advantage of the method is that carbon dioxide used as a solvent can very simply be recirculated for reuse. By reducing the pressure of carbon dioxide the optically pure derivatives of epoxypropionic acid precipitate and can simply be separated from carbon dioxide by allowing the carbon dioxide to evaporate under atmospheric pressure.

It is a further advantage of this method that carbon dioxide does not leave any residue in the final product, as it is the case when using the already known methods. Therefore the method according to this invention advantageously improves the quality of the product.

Carbon dioxide separated from the product is evaporated and, after elevating the pressure and adjusting the temperature, is returned to dissolve a new batch of racemic crude material. Due to the low heat of evaporation of carbon dioxide, the energy needed for recycling the carbon dioxide is only a fraction of the energy needed for redistillation of organic solvents. Therefore the method according to this invention significantly reduces pruduction costs.

One of the benefits of this invention is also that the carbon dioxide used as a solvent is inexpensive, incombustible and non-poisonous. This brings savings in explosion protection of the equipment and buildings and in controlling the hazards of solvent effluents. Thus the improved occupational safety is also an advantage of this method. Plants using carbon dioxide as a solvent do not cause harmful solvent effluents to the environment.

It is characteristic of the method according to this invention that the racemic derivative of epoxypropionic acid is dissolved in carbon dioxide under elevated pressure. The obtained solution is charged to the chromatographic column preferably at a temperature of from 0 to 120°C, most preferably of from 30 to 60°C, and under elevated pressure, preferably of from 100 and 400 bar, and most preferably of from 150 to 300 bar. A continuous eluant flow passes through the chromatographic column. Carbon dioxide is used as the eluant, optionally containing a small amount, preferably 0.1 to 1 weight-%, of a modifier, preferably a low molecular weight alcohol, for instance methanol, ethanol or propanol, or water. The chromatographic column has been packed with solid packing material. The packing material has been prepared by coating suitable particles, preferably silica gel particles, with suitable chiral materials, preferably with cellulose esters or cellulose carbamates.

The composition of the eluate from the chromatographic column is observed with a suitable detector and the eluate is fractionated so that one fraction contains the product, i.e. the desired optical isomer of the derivative of epoxypropionic acid, in the desired purity. The eluate from the chromatographic column can be further fractionated, for example, into two fractions, whereby one fraction can still contain a considerable amount of the desired optical isomer and can be returned to the racemic mixture to be charged to the column. The last fraction contains almost exclusively the non-desired optical isomer from the racemic mixture and is removed.

Batches of the racemic derivatives of epoxypropionic acid dissolved in carbon dioxide are charged to the chromatographic column successively so that the more quickly eluting isomer of the latter batch does not get mixed with the slowest eluting isomer of the previous batch.

The amount of the racemic mixture charged at a time to the column preferably is at least 0.003 g per kg of the filling material of the column.

The pressure of the fractions of the eluate from the chromatographic column is reduced and/or the temperature is elevated so that the derivatives of the epoxypropionic acid dissolved therein precipitate. Preferably the eluate is allowed to expand adiabatically to a pressure of 50 to 60 bar. The precipitated derivatives of epoxypropionic acid are transferred with the eluant to the pressure vessels where the carbon dioxide is evaporated and the product is released to atmospheric pressure.

The carbon dioxide eluant evaporated in the pressure vessels is returned, after purifying and liquifying, if needed, to the inlet of the chromatographic column.

The following examples illustrate the invention.

### EXAMPLE 1

The racemic methyl ester of (p-methoxyphenyl)epoxypropionic acid was dissolved in carbon dioxide in a pressure vessel at 40°C under a pressure of 260 bar. The concentration of the dissolved ester in the carbon dioxide phase was 8 weight-%.

The carbon dioxide solution containing the methyl ester of (p-methoxyphenyl)epoxypropionic acid obtained from the dissolving vessel was passed for ten seconds to the carbon dioxide flow, the temperature of which was 40°C and the pressure 250 bar. The obtained mixture was passed to the chromatographic column filled with silica gel particles coated with cellulose-tris-(3,5-dimethylphenyl)carbamate.

Part of the eluate from the chromatographic column was passed continuously to a flame ionization detector (FID). According to the signal given by the detector, the batch of the methyl ester of racemic (p-methoxyphenyl)epoxypropionic acid charged to the chromatographic column was separated in the column almost completely into two pure enantiomers, which were eluting from the column after 24 minutes and at intervals of 4 minutes.

### EXAMPLE 2

The packing material of the chromatographic column and the conditions in the arrangements described in example 1 were varied. The methyl ester of (p-methoxyphenyl)epoxypropionic acid was still used as the racemic mixture to be separated.

| Test number | Temperature °C | Pressure bar | Filling material of the column | Retention time of first peak min | Interspace between the optical isomer peaks min |
|---|---|---|---|---|---|
| 1 | 120 | 100 | crown ether | 9 | 0 |
| 2 | 120 | 100 | branched polysiloxane | 18 | 0 |
| 3 | 60 | 150 | microcrystalline cellulosetriacetate | 45 | 1 |
| 4 | 40 | 250 | microcrystalline cellulosetriacetate | 10 | 4 |
| 5 | 40 | 200 | cellulose-tris-(3,5-dimethyl-phenyl) carbamate | 40 | 6 |
| 6 | 35 | 300 | cellulose-tris-(p-methylphenyl) carbamate | 22 | 7 |

### EXAMPLE 3

The arrangements described in example 1 were varied with regard to the eluant entering the chromatographic column. Cellulose-tris-(3,5-dimethylphenyl)carbamate was used as packing material of the chromatographic column.

| Test number | Pressure bar | Temperature °C | Retention time of the first eluating enantiomer min | Interspace between the enantiomer peaks min |
|---|---|---|---|---|
| 7 | 180 | 40 | 48 | 8 |
| 8 | 200 | 40 | 40 | 6 |
| 9 | 250 | 40 | 23 | 4 |
| 10 | 300 | 40 | 22 | 3 |
| 11 | 200 | 30 | 22 | 6 |
| 12 | 250 | 30 | 22 | 4 |
| 13 | 200 | 20 | 17 | 0 |

### EXAMPLE 4

The arrangements described in example 1 were varied with regard to the amount of the racemic mixture charged to the chromatographic column.

| Test number | Amount of the charged racemic mixture g/kg of the column's filling material | Retention time of the first eluating enantiomer mins | Interspace between the enantiomer peaks min | Measured resolution Rs |
|---|---|---|---|---|
| 14 | 0.003 | 23 | 4 | 1.44 |
| 15 | 0.01 | 39 | 5 | 0.92 |
| 16 | 0.02 | 21 | 3 | 0.94 |
| 17 | 0.04 | 24 | 3 | 0.68 |
| 18 | 0.08 | 21 | 3 | 0.56 |
| 19 | 0.16 | 23 | 3 | 0.58 |
| 20 | 0.32 | 22 | 2 | 0.38 |
| 21 | 1 | 22 | 1.9 | 0.31 |
| 22 | 5 | 21 | 1.5 | 0.22 |
| 23 | 10 | 21 | 1.6 | 0.18 |

Resolution has been used as a measure in evaluating the experimental results presented in example 4. The measured resolution Rs is the difference between the retention times of the eluting peaks divided by the bottomwidth of the latter eluted peak.

There is a certain dependency between the obtained resolution results and the amount of the charged racemic mixture in the experiments described in example 4. When the resolution is very low, the eluting peaks of optically pure isomers almost overlap and the amount of the pure isomer obtained as product from the charged amount of racemic mixture is small. An economic preparation process requires that the resolution is at least in the range of 0.1 to 0.2.

## Claims

1. A method for preparing optically pure derivatives of epoxypropionic acid having the general formula 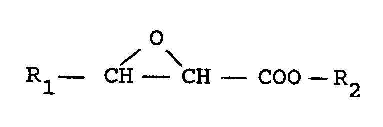 wherein R₁ and R₂, which may be the same or different from each other, are H, an alkyl group or a substituted or unsubstituted phenyl group, from racemic mixtures thereof, comprising the following steps:
a) dissolving the racemic mixture to be separated in carbon dioxide,
b) passing the obtained solution through a chromatographic column containing a chiral material, and
c) separating the desired optical isomer of epoxypropionic acid from the eluant.

2. The method according to claim 1, wherein the eluant essentially consisting of carbon dioxide is passed continuously to the chromatographic column.

3. The method according to any one of claims 1 and 2, wherein the racemic mixture of epoxypropionic acid derivative dissolved in the eluant essentially consisting of carbon dioxide is periodically passed with the continuous eluant flow to the chromatographic column so that the amount of the racemic mixture charged to the column is at least 0.003 g per kg of the packing material of the column.

4. The method according to any one of claims 1 to 3, wherein the eluant essentially consisting of carbon dioxide is passed to the chromatographic column at a temperature of from 0°C to 120°C, preferably of from 30°C to 60°C.

5. The method according to any one of claims 1 to 4, wherein the eluant essentially consisting of carbon dioxide is passed to the chromatographic column under a pressure of from 100 to 400 bar, preferably of from 150 to 300 bar.

6. The method according to any one of claims 1 to 5, wherein the pressure of each fraction of the eluate is reduced to not more than 60 bar so that the derivative of epoxypropionic acid dissolved in each fraction precipitates from the eluate.

7. The method according to any one of claims 1 to 6, wherein the racemic epoxypropionic acid derivative to be separated is an ester of (p-methoxyphenyl)epoxypropionic acid.

8. The method according to any one of claims 1 to 7, wherein the chromatographic column is packed with material made of microcrystallized cellulosetriacetate, cellulose-tris-(3,5-dimethylphenyl)carbamate or cellulose-tris-(p-methylphenyl)carbamate.

## Patentansprüche

1. Verfahren zur Herstellung optisch reiner Derivate von Epoxypropionsäure mit der allgemeinen Formel 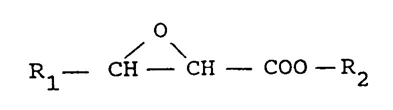 worin R₁ und R₂, die gleich oder verschieden voneinander sein können, für H, eine Alkylgruppe oder eine substituierte oder unsubstituierte Phenylgruppe stehen, aus racemischen Mischungen davon, umfassend die folgenden Stufen:
a) Lösen der aufzutrennenden racemischen Mischung in Kohlendioxid,
b) Leiten der erhaltenen Lösung durch eine Chromatographiesäule, die ein chirales Material enthält, und
c) Abtrennen des gewünschten optischen Isomeren von Epoxypropionsäure vom Elutionsmittel.

2. Verfahren nach Anspruch 1, in dem das Elutionsmittel, das im wesentlichen aus Kohlendioxid besteht, kontinuierlich durch die Chromatographiesäule geleitet wird.

3. Verfahren nach irgendeinem der Ansprüche 1 und 2, in dem die racemische Mischung von Epoxypropionsäure-Derivat, die in dem im wesentlichen aus Kohlendioxid bestehenden Elutionsmittel gelöst ist, mit dem kontinuierlichen Elutionsmittelstrom periodisch so durch die Chromatographiesäule geleitet wird, daß die Menge an racemischer Mischung, mit der die Säule beschickt wird, mindestens 0,003 g pro kg des Packungsmaterials der Säule beträgt.

4. Verfahren nach irgendeinem der Ansprüche 1 bis 3, in dem das im wesentlichen aus Kohlendioxid bestehende Elutionsmittel bei einer Temperatur von 0°C bis 120°C, vorzugsweise von 30°C bis 60°C, zur Chromatographiesäule geleitet wird.

5. Verfahren nach irgendeinem der Ansprüche 1 bis 4, in dem das im wesentlichen aus Kohlendioxid bestehende Elutionsmittel unter einem Druck von 100 bis 400 Bar, vorzugsweise von 150 bis 300 Bar, zur Chromatographiesäule geleitet wird.

6. Verfahren nach irgendeinem der Ansprüche 1 bis 5, in dem der Druck einer jeden Fraktion des Eluats auf nicht mehr als 60 Bar vermindert wird, so daß das Derivat von Epoxypropionsäure, das in jeder Fraktion gelöst ist, aus dem Eluat ausfällt.

7. Verfahren nach irgendeinem der Ansprüche 1 bis 6, in dem das aufzutrennende racemische Epoxypropionsäure-Derivat ein Ester von (p-Methoxyphenyl)epoxypropionsäure ist

8. Verfahren nach irgendeinem der Ansprüche 1 bis 7, in dem die Chromatographiesäule mit einem Material gepackt ist, das aus mikrokristallisiertem Cellulosetriacetat, Cellulose-tris-(3,5-dimethylphenyl)carbamat oder Cellulose-tris-(p-methylphenyl)carbamat hergestellt ist.

## Revendications

1. Procédé pour la préparation de dérivés optiquement purs d'acide époxypropionique ayant la formule générale : 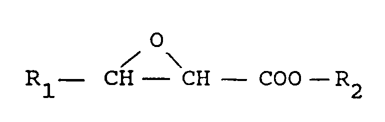 dans laquelle R₁ et R₂ peuvent être identiques ou différents l'un de l'autre, ils sont un atome d'hydrogène, un groupe alkyle ou un groupe phényle substitué ou non substitué, à partir de leurs mélanges racémiques, comprenant les étapes suivantes :
(a) dissolution du mélange racémique à séparer dans du dioxyde de carbone
(b) envoi de la solution obtenue à travers une colonne chromatographique contenant un matériau chiral, et
(c) séparation de l'isomère optique désiré d'acide époxypropionique et de l'éluant.

2. Procédé suivant la revendication 1, dans lequel l'éluant consistant essentiellement en dioxyde de carbone est envoyé en continu dans la colonne chromatographique.

3. Procédé suivant l'une quelconque des revendications 1 et 2, dans lequel le mélange racémique de dérivés d'acide époxypropionique dissous dans l'éluant consistant essentiellement en dioxyde de carbone est envoyé de façon périodique avec le flux d'éluant continu dans la colonne chromatographique de telle sorte que la quantité de mélange racémique chargé dans la colonne représente au moins 0,003 g/kg du matériau de garnissage de la colonne.

4. Procédé suivant l'une quelconque des revendications 1 à 3, dans lequel l'éluant consistant essentiellement en dioxyde de carbone est envoyé dans la colonne chromatographique à une température de 0°C à 120°C, de préférence de 30°C à 60°C.

5. Procédé suivant l'une quelconque des revendications 1 à 4, dans lequel l'éluant consistant essentiellement en dioxyde de carbone est envoyé dans la colonne chromatographique sous une pression de 100 à 400 bars, de préférence de 150 à 300 bars.

6. Procédé suivant l'une quelconque des revendications 1 à 5, dans lequel la pression de chaque fraction de l'éluat est réduite de façon à ne pas dépasser 60 bars pour que le dérivé d'acide époxypropionique dissous dans chaque fraction précipite à partir de l'éluat.

7. Procédé suivant l'une quelconque des revendications 1 à 6, dans lequel le dérivé d'acide époxypropionique racémique à séparer est un ester d'acide p-méthoxyphényléthoxypropionique.

8. Procédé suivant l'une quelconque des revendications 1 à 7, dans lequel la colonne chromatographique est garnie d'un matériau fait de tri-acétate de cellulose microcristallisée, tris-(3,5-diméthylphényl)carbamate de cellulose ou de tris-(p-méthylphényl)carbamate de cellulose.
